(19) Europäisches Patentamt
European Patent Office
Office européen des brevets

(11) Veröffentlichungsnummer: **0 093 905**
**B1**

(12) # EUROPÄISCHE PATENTSCHRIFT

(45) Veröffentlichungstag der Patentschrift: **02.01.85**

(51) Int. Cl.³: **C 07 C 135/02,** C 11 D 1/90 // C11D1/75

(21) Anmeldenummer: **83103841.9**

(22) Anmeldetag: **20.04.83**

(54) **Betain-Aminoxide, Verfahren zu ihrer Herstellung und ihre Verwendung als Tenside.**

(30) Priorität: **24.04.82 DE 3215451**

(43) Veröffentlichungstag der Anmeldung: **16.11.83 Patentblatt 83/46**

(45) Bekanntmachung des Hinweises auf die Patenterteilung: **02.01.85 Patentblatt 85/1**

(84) Benannte Vertragsstaaten: **AT BE CH DE FR GB IT LI NL SE**

(56) Entgegenhaltungen:
**EP - A - 0 080 137**
**DE - A - 2 063 422**
**US - A - 3 265 719**

(73) Patentinhaber: **HOECHST AKTIENGESELLSCHAFT, Postfach 80 03 20, D-6230 Frankfurt am Main 80 (DE)**

(72) Erfinder: **Blaschke, Günter, Dr., Bajuwarenstrasse 36, D-8261 Winhöring (DE)**
Erfinder: **Reng, Alwin, Im Schulzehnten 22, D-6233 Kelkheim (Taunus) (DE)**
Erfinder: **Quack, Jochen Meinhard, Dr., Wilhelm-Reuter-Strasse 16, D-6239 Eppstein/Taunus (DE)**

**EP 0 093 905 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Es ist bereits bekannt, amphotere Tenside mit einer Betain-Gruppe im Molekül oder Aminoxide mit einer Aminoxidgruppe im Molekül allein oder in Kombination mit anionischen Tensiden in Reinigungsmitteln einzusetzen. Aus der DE-AS 12 49 433 ist beispielsweise der Einsatz von Alkyl-Betainen in Reinigungsmitteln bekannt, während Amidoalkyl-Betaine in der DE-AS 11 72 802 als hautverträgliche Badezusätze, in der DE-AS 15 37 218 als keimtötende, die Augen nicht reizende Haarwaschmittel empfohlen werden. Als vorteilhafte, hautfreundliche Tenside werden auch in der DE-OS 20 63 422 Tensidgemische aus Monobetainen und Monoaminoxiden genannt.

Als bisfunktionelle Aminoxide sind die Diamin-diaminoxide aus der US-PS 31 97 509 und US-PS 32 34 139 bekannt. Als bisfunktionelle Dibetaine werden in der DE-AS 21 39 074 in einer Waschmittelzusammensetzung unter anderen Polyaminpolybetaine als amphotere oberflächenaktive Reinigungsmittel beschrieben.

Es hat sich aber gezeigt, daß weder Diamin-diaminoxide noch Diamin-dibetaine den wachsenden Ansprüchen an Reinigungsmittelkomponenten gerecht werden können.

Überraschenderweise wurde nun gefunden, daß amphotere Tenside, die sowohl eine oder mehrere Aminoxidfunktionen und gleichzeitig eine oder mehrere Betainfunktionen in einem Molekül enthalten, bessere anwendungstechnische Eigenschaften aufweisen als die reinen Aminoxide bzw. Betaine oder auch deren Mischungen untereinander.

Gegenstand der Erfindung sind somit gemischte Betain-Aminoxide der Formel

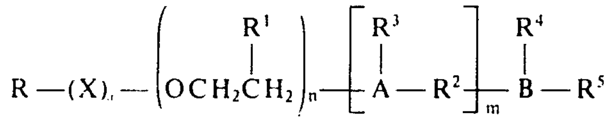

worin

R Alkyl, Alkenyl oder Hydroxyalkyl mit jeweils 8–22, vorzugsweise 12–18 C-Atomen,
$R^1$ Wasserstoff oder Methyl,
$R^2$ Äthylen, Propylen oder 2-Hydroxypropylen,
$R^3$, $R^4$ und $R^5$ gleich oder verschieden sein können und $C_1$–$C_3$-Alkyl oder eine Gruppe der Formel

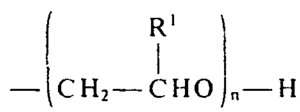

und $R^3$ auch die Gruppe

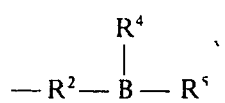

X eine direkte Bindung oder eine Gruppe der Formel

$-CO-$

A und B eine Gruppe der Formeln

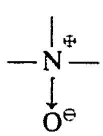

und

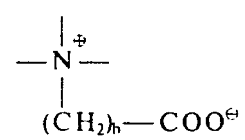

bedeuten, wobei mindestens eine Aminoxid- und mindestens eine Betain-Funktion vorhanden sein muß, a 0 oder 1, b 1, 2 oder 3, n eine Zahl von 0 bis 10 und m 1, 2 oder 3 bedeuten.

Bevorzugt sind solche Verbindungen der obigen Formel, worin $R^1$ Wasserstoff, $R^2$ Propylen oder Hydroxypropylen, $R^3$, $R^4$ und $R^5$ gleich sind und Methyl oder Hydroxyethyl bedeuten, a Null und m 1 oder 2 ist.

Diese Verbindungen werden hergestellt, indem man ein Mol eines Polyamins der Formel

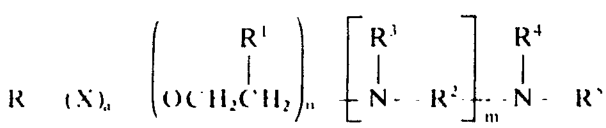

mit 1 bis 3 Mol eines Alkalisalzes einer ω-Halogencarbonsäure der Formel

$$Hal-(CH_2)_n-COOH$$

quaterniert und das so erhaltene Betain anschließend mit Wasserstoffperoxid oxidiert.

Die Herstellung der erfindungsgemäßen Betain-Aminoxide erfolgt aus Polyaminen, die tertiäre Stickstoffatome enthalten. Diese Polyamine sind bereits seit langem bekannt. Sie werden erhalten beispielsweise durch Reaktionen an Alkylaminen oder Alkylpolyoxyethylaminen, die mindestens noch ein reaktives Wasserstoffatom am basischen Stickstoffatom tragen. Solche Reaktionen können sein Cyanalkylierungen mit Acrylnitril mit anschließender Hydrierung, die mehrfach wiederholt werden kann; Aminoalkylierungen mit cyclischen Aminverbindungen wie beispielsweise Aziriden; Umsetzung mit $\omega$-Halogenalkylnitrilen oder $\omega$-Halogenamine, wie beispielsweise Chloracetonitril, Chlorethyldimethylamin oder Chlorethyldiethanolamin; Epoxidierungen mit Verbindungen wie Ethylenoxid, Propylenoxid, Glycid oder Epichlorhydrin mit nachfolgender Ammonolyse oder Aminolyse. Die so erhaltenen Polyamine werden mit üblichen Alkylierungsmitteln wie Alkylhalogeniden, Formaldehyd, Ethylenoxid, Propylenoxid oder deren Gemischen zu den tertiären Polyaminen umgesetzt (s. Houben–Weyl, Bd. 11/1ff. oder S. J. Gutcho, Surfactants and Sequestrants, Chem. Techn. Rev. 89). Als Ausgangsamine können beispielsweise folgende Amine eingesetzt werden, die teilweise auch als Handelsprodukte verfügbar sind:

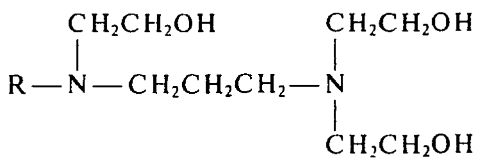
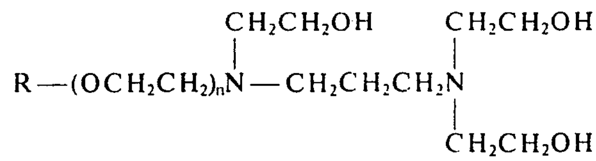

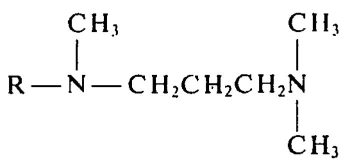
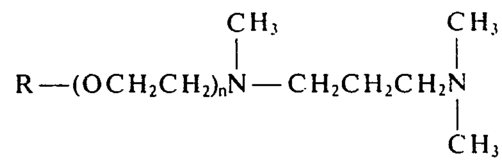

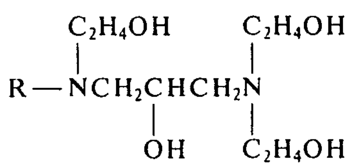
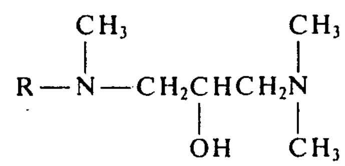

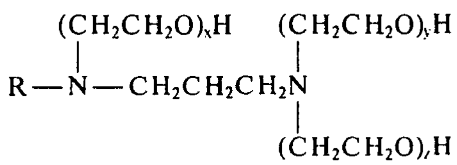
x + y + z = 3-10

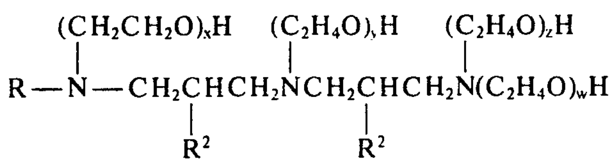
x + y + z + w = 4-20
$R^2$ = H oder OH

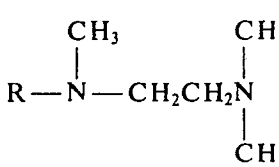
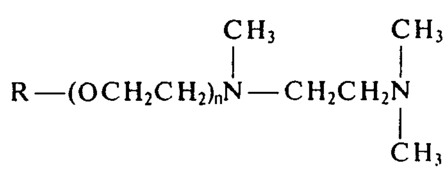

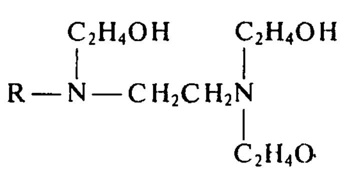
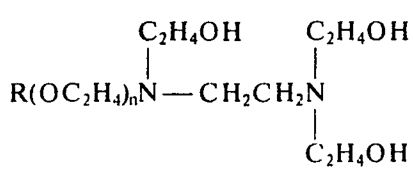

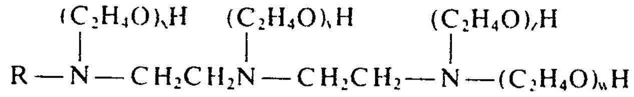

$z + x + y + w = 4\text{–}20$

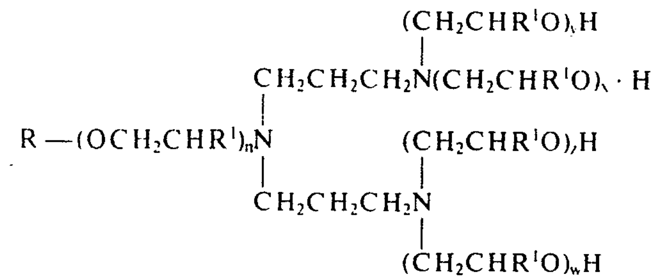

$n = 0\text{–}10$

$x + y + z + w = 4\text{–}20$

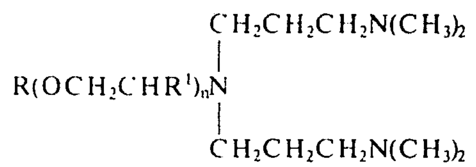

Bei dem Rest R in diesen Ausgangsverbindungen und entsprechend auch in den Endprodukten handelt es sich um langkettige Alkyl-, Alkenyl- oder Hydroxialkylgruppen, vorzugsweise solche, die sich von natürlich vorkommenden Fettsäuren, aus geraden oder verzweigten Alkylketten, die sich aus dem Ziegler-Prozeß (Ethylenaufbaualkohole) oder aus der Oxosynthese ableiten. Dementsprechend können die Ausgangsprodukte und die Endprodukte Gemische darstellen mit unterschiedlicher Bedeutung des Restes R in Abhängigkeit von der Zusammensetzung des jeweiligen Fettalkyl-Gemisches. Beispiele für solche Gemische, die sich von natürlich vorkommenden Fettsäuren ableiten, sind Talgfettalkyl oder Cocosfettalkyl.

Aus den tertiären Polyaminen werden die erfindungsgemäßen Verbindungen durch Quaternisierung mit einem Alkalisalz einer $\omega$-Halogencarbonsäure, vorzugsweise mit den Natriumsalzen von Chloressigsäure, Chlorpropionsäure, Bromessigsäure oder Chlor-n-Buttersäure. Anstelle dieser Alkalisalze kann man die Reaktion auch mit den entsprechenden freien Säuren unter Zugabe von Alkalihydroxid durchführen. Die Quaternierung verläuft in wäßriger Lösung bei Temperaturen von ca. 70–100°C. Das Mengenverhältnis von Polyamin und Halogencarbonsäuresalz wird so gewählt, daß mindestens ein Stickstoffatom im Molekül quaterniert wird. Mindestens ein Stickstoffatom im Molekül darf jedoch nicht quaterniert werden, sondern muß für die Einführung der Aminoxid-Funktion frei bleiben. Demgemäß beträgt das molare Verhältnis von Halogencarbonsäure-Salz zu Polyamin 1 : 1 bis 3 : 1.

Es ist bekannt, daß die Geschwindikeit der Quaternisierungsreaktion von tertiären Aminen mit $\omega$-Halogencarbonsäuren bzw. dessen Alkalisalzen von der Basizität, Reaktivität und der sterischen Umgebung der tertiären Stickstoffatome abhängig ist, wobei die Geschwindigkeit der Umsetzung und Umsetzungsgrad von beispielsweise Chloressigsäure-Natrium mit tertiärem Amin in folgender Reihenfolge abnimmt:

$$R{-}N(CH_3)_2 > R{-}N(C_2H_4OH)_2 \gg R_2{-}N{-}CH_3 > R_2N(C_2H_4OH) \gg R_3N$$

Nach dieser Reihe werden bevorzugt endständige tertiäre Aminogruppen mit $\omega$-Halogencarbonsäure-Alkalisalz quaternisiert, innere tertiäre Aminogruppen werden nur in untergeordneter Weise quaternisiert, da sie einer $R_2N(CH_3)$- oder $R_2N(C_2H_4OH)$-Gruppe entsprechen.

Als Zwischenstufe erhält man so ein Alkyl- oder Alkylpolyoxalkylpolyaminoalkyl-betain, vorzugsweise als 30–40%ige Lösung in Wasser. Der verbleibende, nicht quaternisierte tert. Stickstoff des als Zwischenstufe gebildeten Aminobetains wird anschließend mit 35- oder 70%igem Wasserstoffperoxid, das in 5 bis 10%igem molarem Überschuß, bezogen auf freie tert. Aminogruppen, angewandt wird, bei einer Temperatur von 60–90°C zu den erfindungsgemäßen polyfunktionellen Betain/Aminoxiden oxidiert. Vorteilhafterweise stellt man durch geeignete Wahl des Wassergehaltes bei der letzten Reaktionsstufe die erfindungsgemäßen Betain/Aminoxide als 30–40%ige wäßrige Einstellungen her. Neben den erfindungsgemäßen Betain-Aminoxiden kann das Reaktionsprodukt auch noch unterschiedliche Mengen solcher Polyamine enthalten, die ausschließlich Betain-Funktionen oder ausschließlich Aminoxid-Funktionen tragen. Der Anteil dieser Verbindungen ist jedoch unkritisch für die praktische Anwendung.

Die erfindungsgemäßen Betain-Aminoxide werden verwendet als Tenside in den bekannten und üblichen Reinigungsmitteln, wie beispielsweise Waschmittel jeder Art für das Haar und den Körper, Haushaltreinigungsmittel, Waschmittel für Textilien, Geschirrspülmittel, Autoreinigungsmittel und sonstige technische Reinigungsmittel. Der Gehalt an Betain-Aminoxid in diesen Reinigungsmitteln liegt innerhalb der hierbei üblichen Grenzen, d. h. etwa zwischen 1 und 50, vorzugsweise 5 und 15 Gew.-%. Die Art

und Menge der sonstigen Komponenten in diesen Reinigungsmitteln ist üblich und bekannt und bedarf daher keiner weiteren Erläuterungen.

Der Vorteil der erfindungsgemäßen Produkte gegenüber den einheitlichen Polyamin-polybetainen und Polyamin-polyoxiden liegt darin, daß das Schaumverhalten, die Schaumstabilität, die Viskosität, die Beeinflußbarkeit der Verdickung mit Elektrolyten und Alkylsulfaten oder Ethersulfaten, das Kälteverhalten, das Netzvermögen und die Haarkonditioniereffekte beim Einsatz in Shampoos verbessert werden.

## Beispiel 1

In einem Kolben mit Rückflußkühler, Thermometer, Rührer und Dosiergefäß werden 170,4 g (0,4 Mol) Laurylpropylendiamin-tris-oxethylat der Formel

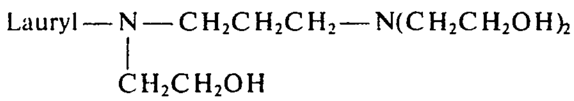

(Lauryl = 73% $C_{12}$ und 23% $C_{14}$) sowie 318,9 g Wasser vorgelegt und unter Rühren auf 90°C erwärmt. Bei dieser Temperatur werden dann innerhalb einer Stunde 46,6 g (0,4 Mol) Natriumchloracetat in 108,7 g Wasser zugegeben und 12 h bei 95°C nachgerührt. Anschließend werden 23,3 g (0,48 Mol) 70%iges Wasserstoffperoxid zugegeben und bei 70°C weitere 8 h gerührt. Man erhält das erfindungsgemäße Betain-Aminoxid 30%ig in wäßriger Lösung.

Der Umsatz wird durch die Bestimmung der Aminzahl, des Gehalts an Aminoxid und durch Vergleich von organisch gebundenem und gesamten Chlorgehalt nach gängigen Analysenmethoden kontrolliert.

## Beispiel 2

286,3 g (0,7 Mol) Cocosfettaminopropylamin-tris-oxethylat der Formel

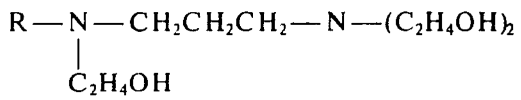

(R = 6% $C_8$; 6% $C_{10}$; 54% $C_{12}$; 18% $C_{14}$; 8% $C_{16}$ und 8% $C_{18}$) und 530 g Wasser werden mit 81,6 g Natriumchloressigsäure in 190,3 g Wasser versetzt und 12 h bei 95°C gerührt. Anschließend wird mit 40,7 g 70%igem Wasserstoffperoxid 8 h bei 70°C oxidiert. Man erhält das Betain-Aminoxid als 30%ige Lösung in Wasser.

## Beispiel 3

619,0 g (1 Mol) Cocosalkylpentaoxethylaminopropylamin-tris-oxethylat der Formel

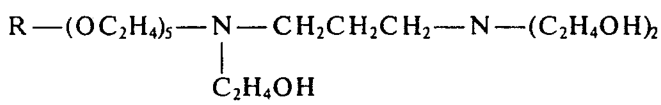

(Zusammensetzung von R wie im Beispiel 2) und 1519,4 g Wasser werden mit 116,5 g Natriumchloracetat (1 Mol) 12 h lang bei 95°C und anschließend mit 58,3 g 70%igem Wasserstoffperoxid zum entsprechenden Betain-Aminoxid umgesetzt.

## Beispiel 4

550,0 g (1 Mol) Talgfettalkyldipropylentriamin-tetrakis-oxethylat der Formel

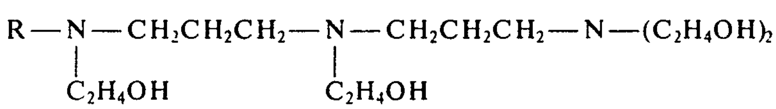

(R = $C_{16}H_{33}$-Alkyl und $C_{18}H_{37}$-Alkyl) werden mit 1329,9 g Wasser mit 233,0 g (2 Mol) Natriumchloracetat bei 95°C 12 h lang und anschließend mit 116,6 g (2 Mol) 70%igem Wasserstoffperoxid 8 h lang bei 70°C umgesetzt.

Man erhält ein statistisches Gemisch aus Bisbetain-Aminoxid und Betain-Bisaminoxid als 30%ige Emulsion in Wasser.

## Beispiel 5

366 g (1 Mol) Cocosalkylamino-2-hydroxypropylamino-tris-oxethylat der Formel

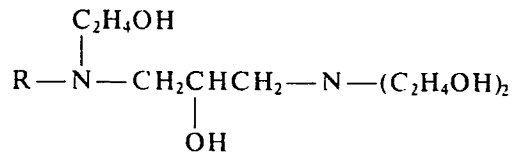

(Zusammensetzung von R wie im Beispiel 2) und 929,1 g Wasser werden mit 122,3 g (1,05 Mol) Natriumchloracetat bei 95°C 12 h lang umgesetzt und anschließend mit 68,0 g (1,4 Mol) 70%igem Wasserstoffperoxid zum Betain-Aminoxid umgesetzt (30%ig in Wasser).

## Beispiel 6

308,0 g (1 Mol) Cocosalkylmethylamino-2-hydroxypropyl-dimethylamin der Formel

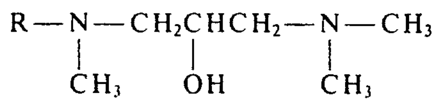

(Zusammensetzung von R wie im Beispiel 2) und 793,7 g Wasser werden mit 128,2 g (1,1 Mol) $ClCH_2COONa$ bei 95°C 8 h und anschließend mit 53,4 g (1,1 Mol) 70%igem $H_2O_2$ bei 70°C umgesetzt. Man erhält das Betain-Aminoxid als 30%ige Lösung in Wasser.

Die nachfolgend aufgeführten Anwendungsbeispiele zeigen die Einsatzmöglichkeiten der Betain-Aminoxide in Haar- und Körperreinigungsmitteln. Die Mengen- und Prozentangaben in den Beispielen beziehen sich, sofern nicht anders erwähnt, jeweils auf das Gewicht.

Haarshampoo mit Avivage-Effekt

| | |
|---|---|
| Betain-Aminoxid, hergestellt nach Beispiel 2 | 15,00% |
| Polyethylenglykol-6000-distearat | 5,20% |
| Parfümöl | 0,30% |
| Formaldehyd | 0,05% |
| Wasser | ad 100,00% |

Haarshampoo

| | |
|---|---|
| Betain-Aminoxid, hergestellt nach Beispiel 2 | 12,00% |
| Hydroxyethylcelluloseether | 1,40% |
| Parfümöl | 0,30% |
| Formaldehyd | 0,05% |
| Wasser | ad 100,00% |

Saures Shampoo

| | |
|---|---|
| Betain-Aminoxid, hergestellt nach Beispiel 1 | 15,00% |
| Citronensäure | 0,30% |
| Parfümöl | 0,10% |
| Konservierungsmittel, Farbstoffe, Wasser | ad 100,00% |

Antischuppenshampoo

| | |
|---|---|
| Betain-Aminoxid, hergestellt nach Beispiel 3 | 5,00% |
| Palmkernfettsäuremethyltaurid-Natriumsalz | 6,00% |
| Stearinsäuremethyltaurid-Natriumsalz | 4,00% |
| Lauroylsarkosid-Natriumsalz | 2,00% |
| 2-Mercaptopyridin-N-oxid-Zinksalz | 0,50% |
| Parfümöl | 0,20% |
| Wasser, Farbstoffe | ad 100,00% |

Shampoo für fettige Haare

| | | |
|---|---|---|
| Betain-Aminoxid, hergestellt nach Beispiel 3 | | 7,00% |
| sekundäres Alkansulfat-Natriumsalz (Alkanrest $C_{13}$–$C_{17}$) | | 5,00% |
| $\alpha$-Olefinsulfonat ($C_{14}$–$C_{16}$)-Natriumsalz | | 2,00% |
| Laurylsulfat-Natriumsalz | | 2,00% |
| Wasser, Konservierungsmittel, Farbstoffe | ad | 100,00% |

Duschbad

| | | |
|---|---|---|
| Betain-Aminoxid, hergestellt nach Beispiel 4 | | 12,00% |
| Lauryltetraglykolethersulfosuccinat-Dinatriumsalz | | 3,00% |
| Hydroxyethylcelluloseether | | 1,20% |
| Parfümöl | | 0,10% |
| Cocosfettsäuremonoethanolamid | | 0,80% |
| Wasser, Konservierungsmittel, Farbstoffe | ad | 100,00% |

Schaumbad

| | | |
|---|---|---|
| Betain-Aminoxid, hergestellt nach Beispiel 5 | | 5,00% |
| Lauryldiglykolethersulfat-Natriumsalz | | 20,00% |
| sekundäres Alkansulfonat-Natriumsalz (Alkanrest $C_{13}$–$C_{17}$) | | 5,00% |
| Cocosfettsäurediethanolamid | | 2,00% |
| Parfümöl | | 0,40% |
| Natriumchlorid | | 3,00% |
| Wasser, Konservierungsmittel, Farbstoffe | ad | 100,00% |

Schaumverhalten

Zur Demonstration des verbesserten Schaumverhaltens der erfindungsgemäßen gemischten Betain-Aminoxide wurden Vergleichsversuche gemacht gegenüber den analogen Bis-betainen und Bisaminoxiden gemäß US-PS 31 97 509 und DE-AS 21 39 074. Da in der Praxis aus kommerziellen Gründen häufig Kombinationen von zwei oder mehr Tensiden benutzt werden, wurde hier eine Mischung aus 7 Teilen Alkyl-diglykoläthersulfat-Natrium (R = 75–70% $C_{12}$ und 25–30% $C_{14}$) sowie jeweils 3 Teilen Betain-Aminoxid nach Beispiel 2 bzw. 3 Teilen des analogen Bis-betains und Bisaminoxids genommen. Die Messung der Schaumhöhe in Millimeter nach der Methode von Ross-Miles bei einer Wasserhärte von 20°dH ergab dabei folgende Werte:

| Tensidkonz. in % | Betain-Aminoxid nach Beispiel 2 | Bis-Betain | Bis-Aminoxid |
|---|---|---|---|
| 1,0 | 270 | 260 | 245 |
| 0,3 | 255 | 240 | 235 |
| 0,1 | 245 | 230 | 215 |
| 0,07 | 225 | 210 | 195 |
| 0,03 | 180 | 165 | 145 |
| 0,01 | – | 105 | – |
| 0,006 | 95 | 60 | 25 |
| 0,002 | 30 | 25 | 15 |

Die Werte zeigen die verbesserte Wirkung der gemischten Betain-Aminoxide.

**Patentansprüche**

1. Betain-Aminoxide der Formel

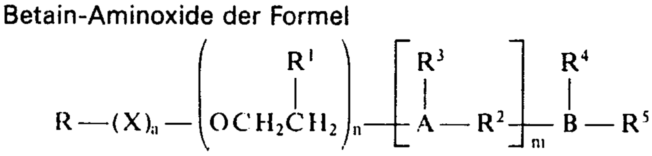

worin

R Alkyl, Alkenyl oder Hydroxyalkyl mit jeweils 8–22, vorzugsweise 12–18 C-Atomen,

$R^1$ Wasserstoff oder Methyl,

$R^2$ Äthylen, Propylen oder 2-Hydroxypropylen,

$R^3$, $R^4$ und $R^5$ gleich oder verschieden sein können und $C_1$–$C_3$-Alkyl oder eine Gruppe der Formel

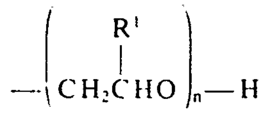

und $R^3$ auch eine Gruppe der Formel

$$-R^2-BR^4R^5$$

X eine direkte Bindung oder eine Gruppe der Formel

$$-CO-$$

A und B eine Gruppe der Formeln

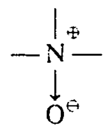

und

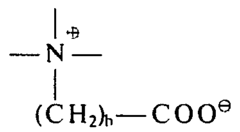

bedeuten, wobei mindestens eine Aminoxid- und mindestens eine Betain-Funktion vorhanden sein muß, a 0 oder 1, b 1, 2 oder 3, n eine Zahl von 0 bis 10 und m 1, 2 oder 3 bedeuten.

2. Betain-Aminoxide nach Anspruch 1, wobei $R^1$ Wasserstoff, $R^2$ Propylen oder 2-Hydroxypropylen, $R^3$, $R^4$ und $R^5$ gleich sind und Methyl oder Hydroxyethyl oder $R^3$ eine Gruppe der Formel

$$-R^2-BR^4R^5$$

bedeuten, a Null und m 2 ist.

3. Verfahren zur Herstellung der Betain-Aminoxide nach Anspruch 1, dadurch gekennzeichnet, daß man ein Mol eines Polyamins der Formel

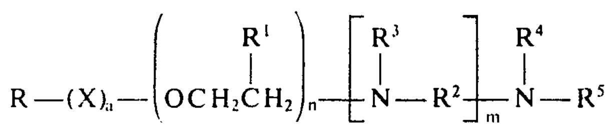

mit 1 bis 3 Mol eines Alkalisalzes einer ω-Halogencarbonsäure der Formel

$$Hal-(CH_2)_b-COOH$$

quaterniert und das so erhaltene Betain anschließend mit Wasserstoffperoxid oxidiert.

4. Verwendung der Betain-Aminoxide nach Anspruch 1 als Tenside in Reinigungsmitteln.

## Claims

1. Betaine-amine oxides of the formula

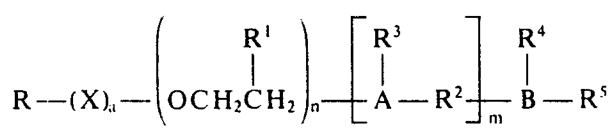

in which

R denotes alkyl, alkenyl or hydroxyalkyl, each of which has 8–22, preferably 12–18 carbon atoms,

$R^1$ denotes hydrogen or methyl,

$R^2$ denotes ethylene, propylene or 2-hydroxypropylene,

$R^3$, $R^4$ and $R^5$ can be identical or different and denote $C_1$–$C_3$-alkyl or a group of the formula

$$-\left(CH_2\overset{R^1}{\overset{|}{C}}HO\right)_n-H$$

and

$R^3$ also denotes the group of the formula

$$-R^2-BR^4R^5$$

X denotes a direct bond or a group of the formula

$$-CO-$$

A and B denote a group of the formula

$$-\overset{|}{\underset{\underset{O^{\ominus}}{\downarrow}}{N^{\oplus}}}- \quad \text{and} \quad -\overset{|}{\underset{\underset{(CH_2)_b-COO^{\ominus}}{|}}{N^{\oplus}}}- .$$

it being necessary for at least one amine oxide group and at least one betaine group to be present, a denotes 0 or 1, b denotes 1, 2 or 3, n denotes a number from 0 to 10 and m denotes 1, 2 or 3.

2. A betaine-amine oxide as claimed in claim 1, in which $R^1$ denotes hydrogen, $R^2$ denotes propylene or 2-hydroxypropylene, $R^3$, $R^4$ and $R^5$ are identical and denote methyl or hydroxyethyl or $R^3$ denotes a group of the formula

$$-R^2-BR^4R^5$$

a is zero and m is 2.

3. The process for the preparation of a betaine-amine oxide as claimed in claim 1, which comprises quaternizing one mole of a polyamine of the formula

$$R-(X)_a-\left(OCH_2\overset{R^1}{\overset{|}{C}}H_2\right)_n-\left[\overset{R^3}{\overset{|}{N}}-R^2\right]_m-\overset{R^4}{\overset{|}{N}}-R^5$$

with 1 to 3 moles of an alkali metal salt of an $\omega$-halogenocarboxylic acid of the formula

$$Hal-(CH_2)_b-COOH$$

and then oxidizing the resulting betaine with hydrogen peroxide.

4. Method of use of a betaine-amine oxide as claimed in claim 1 as a surfactant in cleansing agents.

**Revendications**

1. Bétaïne/oxydes d'amines de formule:

$$R-(X)_a-\left(OCH_2\overset{R^1}{\overset{|}{C}}H_2\right)_n-\left[\overset{R^3}{\overset{|}{A}}-R^2\right]_m-\overset{R^4}{\overset{|}{B}}-R^5$$

dans laquelle

R représente un groupe alkyle, alcényle ou hydroxyalkyle ayant à chaque fois 8 à 22, de préférence 12 à 18, atomes de carbone,

$R^1$ est un atome d'hydrogène ou un groupe méthyle,

$R^2$ représente un groupe éthylène, propylène ou hydroxy-2 propylène,

$R^3$, $R^4$ et $R^5$ peuvent être identiques ou différents et représentent un groupe alkyle en $C_1$ à $C_3$ ou un groupe de formule:

$$-(CH_2CHO)_n-H$$

ou

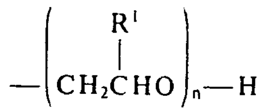

et

$R^3$ pouvant aussi représenter un groupe de formule

$$—R^2—BR^4R^5$$

X étant une liaison directe ou représentant un groupe de formule:

$$—CO—$$

A et B représentant un groupe répondant aux formules:

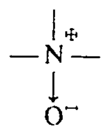 et 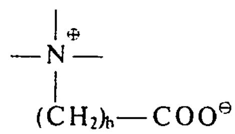

avec présence obligatoire d'au moins une fonction oxyde d'amine et d'au moins une fonction bétaïne, a vaut 0 ou 1, b vaut 1, 2 ou 3, n est un nombre valant 0 à 10 et m est un nombre valant 1, 2 ou 3.

2. Bétaïne/oxydes d'amines selon la revendication 1, dans lesquels $R^1$ représente un atome d'hydrogène, $R^2$ représente un groupe propylène ou hydroxy-2 propylène, $R^3$, $R^4$ et $R^5$ sont identiques et représentent un groupe méthyle ou hydroxyéthyle ou bien $R^3$ est un groupe de formule

$$—R^2—BR^4R^5$$

a est nul et m vaut 2.

3. Procédé de préparation des betaïne/oxydes d'amines selon la revendication 1, caractérisé en ce qu'on quaternise une polyamine de formule:

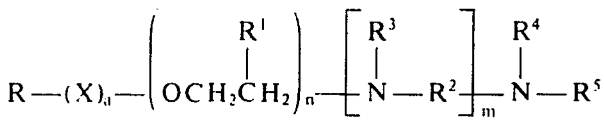

avec 1 à 3 moles d'un sel alcalin d'un acide halogéno-oméga carboxylique de formule:

$$Hal—(CH_2)_b—COOH$$

et l'on soumet ensuite la bétaïne ainsi obtenue à une oxydation par du peroxyde d'hydrogène.

4. Utilisation des bétaïne/oxydes d'amines selon la revendication 1 comme tensio-actifs dans des produits de nettoyage.